# EUROPÄISCHE PATENTANMELDUNG

(11) **EP 0 600 369 A2**
(43) Veröffentlichungstag der Anmeldung: **08.06.1994**
(21) Anmeldenummer: 93118984.9
(22) Anmeldetag: 25.11.1993
(51) Int. Cl.: A61B 5/03

(54) **Pneumatischer Sensor für den intrakraniellen Druck**

(30) Priorität: 30.11.1992 PL 296806
(71) Anmelder: FEHLING MEDICAL AG, D-63791 Karlstein (DE)
(72) Erfinder: Werszko, Miroslaw, Dr., PL-50-350 Wroclaw (PL); Wronski, Jerzy, Dr., PL-50-350 Wroclaw (PL); Morawski, Miroslaw, PL-50-350 Wroclaw (PL)
(74) Vertreter: Patentanwälte Westphal, Buchner, Mussgnug Neunert, Göhring

(57) **Zusammenfassung**

Ein pneumatischer Sensor zur Messung des intrakraniellen Druckes weist einen flachen Kunststoffkörper (1) mit einer kreisförmigen Kammer (6) auf, die durch eine Gummimembran (5) verschlossen ist. Druckluft wird über ein Loch im Boden der Kammer (6) in die Kammer (6) geleitet und entweicht über einen Öffnungsstutzen und einen zwischen dem Öffnungsstutzen (3) und der Gummimembran (5) gebildeten Spalt (7) in die Atmosphäre. Die Gummimembran (5) stellt sich auf eine Lage ein, in welcher der durch die zugeführte Druckluft in der Kammer (6) aufgebaute Druck dem von außen auf die Gummimembran (5) wirkenden intrakraniellen Druck entspricht.

## Beschreibung

Die Erfindung betrifft einen pneumatischen Sensor für den intrakraniellen Druck mit einem Kunststoffkörper in der Form einer flachen Dose und mit einer Meßeinheit, die mit einer Überwachungseinrichtung verbunden ist. Um den Druck bei menschlichen Patienten epidural zu messen, wird der Sensor durch eine Trephine-Öffnung zwischen den Schädel und die Dura mater (harte Hirnhaut) eingeführt.

Aus dem Artikel "Non invasive techniqe of intracranial pressure monitoring" in der Zeitschrift "Kinderchirurgie" No. 31/4, Dezember 1980, Seiten 348 bis 352, ist ein pneumatischer Sensor für den intrakraniellen Druck bekannt, bei welchem der Sensorkörper die Form einer flachen Dose hat. Die eine flache Wand dieses Körpers ist eine Gummimembran, während die übrigen Wände aus Kunststoff bestehen. Der Innenraum des Körpers ist über eine kleine flexible Leitung und drei Lichtwellenleiter mit einer speziellen Überwachungseinrichtung verbunden. Über die flexible Leitung wird dem Inneren des Sensors Druckluft eines geeigneten Druckes zugeführt, wobei die Lichtwellenleiter die neutrale Lage der Membran feststellen und die Information liefern, daß der über die Kunststoffleitung zugeführte und auf die Innenseite der Sensormembran wirkende Druck gleich dem intrakraniellen Druck ist, der auf die Außenseite dieser Membran wirkt. Die wesentlichen Nachteile bei der Verwendung dieses Sensors ergeben sich aus dem komplizierten System des Ausgleichs der Auslenkungen der Membran aus der neutralen Lage, aus der Empfindlichkeit der Meßeinheit gegen Stöße, aus der schwierigen Sterilisation und aus der Notwendigkeit, den Sensor ausschließlich mit einer speziellen Überwachungseinrichtung zu betreiben, die von dem in der Zeitschriftenveröffentlichung angegebenen Hersteller geliefert wird.

Weiter ist aus dem Artikel "Epidural intracranial pressure monitoring" in dem Buch "Intracranial Pressure V", Springer Verlag, Berlin - Heidelberg, 1983, Seiten 85 bis 88, ein Sensor bekannt, dessen Körper aus Polyäthylen besteht und die Form einer flachen Dose aufweist, deren eine Wand eine auf den Körper geklebte Gummimembran ist. Der Innenraum des Körpers ist mit zwei flexiblen kleinen Leitungen, von denen eine mit einer Drahteinlage verstärkt ist, mit einer speziellen Überwachungseinrichtung verbunden. Über eine Leitung wird dem Inneren des Körpers Druckluft mit konstanter Durchflußrate zugeführt, während über die zweite Leitung, deren Austritt durch die Sensormembran abgedeckt werden kann, Luft aus dem Sensorinneren gesaugt wird. Der auf die Sensormembran von der Außenseite wirkende intrakranielle Druck deckt den Austritt der absaugenden Leitung ab, wodurch der Druck unter der Membran ansteigt, bis er gleich dem intrakraniellen Druck wird. Der Druck der dem Sensor von der Überwachungseinrichtung zugeführten Luft ist ein Maß des intrakraniellen Druckes. Die wesentlichen Schwierigkeiten bei der Verwendung dieses Sensors ergeben sich aus der Anpassung des Sensors ausschließlich an eine spezielle Überwachungseinrichtung, die durch den in der Veröffentlichung angegebenen Hersteller geliefert wird und die zwei Druckquellen aufweisen muß, nämlich einen Druckwert über dem Atmosphärendruck und einen Druckwert unter dem Atmosphärendruck.

Aus dem US-Patent 3 863 504 ist weiter eine Vorrichtung zum Messen des Druckes eines Fluids bekannt, welches gegen einen direkten Kontakt mit einem Manometer geschützt werden muß. Die Vorrichtung hat die Form einer Kammer aus einem durchsichtigen Material und ist durch eine nachgiebige Membran in einen oberen und einen unteren Teil getrennt. Der untere Teil der Kammer ist mit dem Fluid verbunden, dessen Druck gemessen werden soll. Der obere Teil der Kammer ist über eine Leitung und eine Dreiweg-Verzweigung mit einem Manometer und einer mit einem neutralen Gas gefüllten Spritze verbunden. Der Druck, der mittels der Spritze erzeugt wird und der durch das Manometer angezeigt wird, wenn sich die durch die durchsichtige Wand der Kammer beobachtete Membran in horizontaler Lage befindet, stellt ein Maß für den Fluiddruck dar.

Der wesentliche Nachteil dieser Vorrichtung besteht darin, daß sie sich nicht für das Messen des intrakraniellen Druckes eignet, weil die Zerebrospinal-Flüssigkeit durch die Kammer fließen müßte. Außerdem ist die Vorrichtung nicht für die epidurale Messung des intrakraniellen Druckes geeignet, d.h. für eine Messung ohne Aufschneiden der Dura mater.

Der Erfindung liegt die Aufgabe zugrunde, die Nachteile der bekannten Vorrichtung zu vermeiden.

Diese Aufgabe wird erfindungsgemäß gelöst durch eine Vorrichtung gemäß Anspruch 1.

Erfindungsgemäß ist eine im Inneren des Polyäthylen-Sensorkörpers gebildete Kammer mit der Atmosphäre verbunden über einen Öffnungsstutzen, der sich in der Mitte der Kammer befindet, und über eine flexible Leitung, deren eines Ende in den Sensorkörper eingesetzt ist, und ist mit einer Druckluftquelle verbunden über ein Loch in dem Kammerboden und eine flexible Leitung, deren eines Ende ebenfalls in den Polyäthylen-Sensorkörper eingesetzt ist. Die Luftkammer wird gebildet durch eine runde Ausnehmung in dem Sensorkörper und durch eine Gummimembran, die mit einem ringförmigen Rand abdichtend in eine Rille des Polyäthylen-Sensorkörpers eingesetzt ist. Der erfindungsgemäße Sensor zeichnet sich aus durch seinen einfachen Aufbau, der Verbindungen der Bauteile durch Verkleben und ein Verstärken dieser Bauteile durch Draht vermeidet. Für die Verbindung des Sensors mit der Überwachungseinrichtung ist nur eine flexible Leitung vorgesehen, die Druckluft eines einzigen Druckwertes zuführt. Der Sensor eignet sich für den Anschluß an eine typische Überwachungseinrichtung, wie sie in jedem Krankenhaus verwendet wird, z.B. zum Anschluß an ein Blutdruck-Meßgerät. Ein weiterer Vorteil des Sensors besteht darin, daß er mit Druckgas, wie Luft, Sauerstoff oder Stickstoff, aus einer Flasche gespeist werden kann, so daß keine Betriebsunterbrechungen auftreten können aufgrund von Ausfällen der elektrischen Stromversorgung.

Nachfolgend wird die Erfindung anhand eines Ausführungsbeispiels unter Bezugnahme auf die beigefügte Zeichnung näher erläutert. Es zeigen :

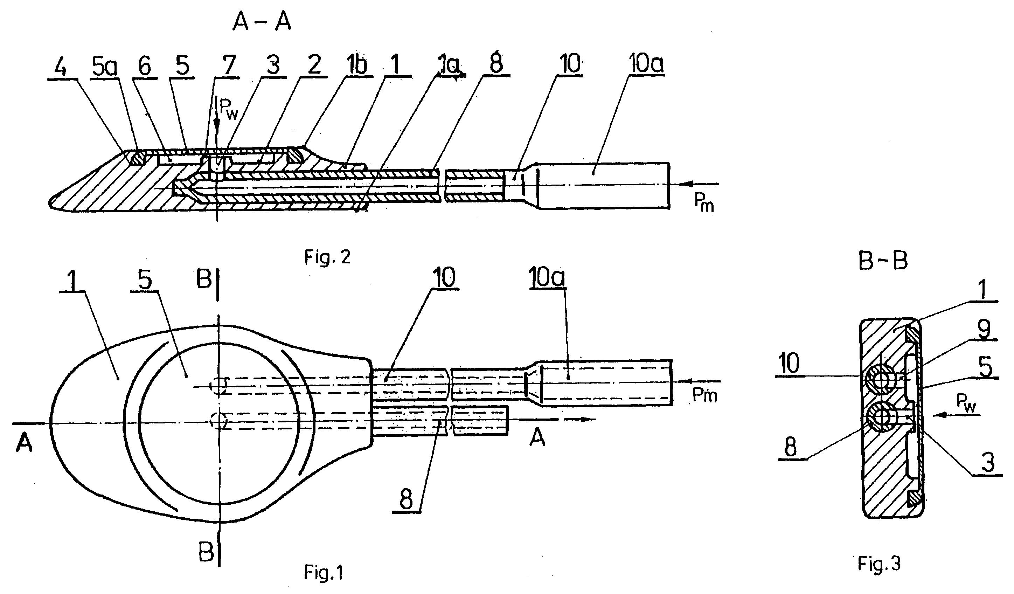

- Figur 1: Eine Draufsicht auf einen Sensor,
- Figur 2: einen Schnitt des Sensors gemäß der Linie A-A in Figur 1 und
- Figur 3: einen Schnitt des Sensors längs der Linie B-B in Figur 1.

Der Sensor weist einen Körper 1 aus Polyäthylen auf, der die Form einer Dose mit einer ebenen Grundfläche 1a und einem ebenen Oberteil 1b aufweist. In dem oberen Teil 1b ist eine runde Ausnehmung 2 ausgeformt, in deren mittlerem Bereich sich ein Öffnungsstutzen 3 befindet. An dem Rand der kreisförmigen Ausnehmung 2 befindet sich eine ringförmige Rille 4 in dem Körper 1, in welche ein ringförmiger Rand 5a einer Gummimembran 5 abdichtend eingesetzt ist. Die Membran 5 deckt die kreisförmige Ausnehmung 2, mit welcher sie eine Luftkammer 6 bildet, dicht ab und bildet mit einer ebenen Stirnfläche des Öffnungsstutzens 3 einen Spalt 7. Die Luftkammer 6 ist mit der Atmosphäre über den Öffnungsstutzen 3 und eine flexible Leitung 8 verbunden, deren eines Ende abgedichtet in den Polyäthylen-Körper 1 eingesetzt ist. Neben dem Öffnungsstutzen 3 ist in dem Boden der Luftkammer 6 ein Loch 9 vorgesehen, das mit einer flexiblen Leitung 10 in Verbindung steht, deren eines Ende abgedichtet in den Körper 1 eingesetzt ist und deren anderes Ende einen Ansatz 10a für den Anschluß an eine Druckluftquelle aufweist.

Zur Messung des intrakraniellen Druckes wird der Sensor nach einer Kraniotomie oder durch eine Trephine-Öffnung so zwischen den Schädelknochen und die Dura mater eingeführt, daß die Gummimembran 5 mit der Dura mater in Berührung ist. Die flexible Leitung 10 wird mittels ihres Ansatzes 10a mit der Druckluftquelle verbunden, vorzugsweise mit einer in der Überwachungseinrichtung angeordneten Druckluftquelle. Komprimierte Luft mit einem Druck Pm wird über die flexible Leitung 10 und das Loch 9 in die Kammer 6 zugeführt, aus welcher sie über den Spalt 7, den Öffnungsstutzen 3 und die flexible Leitung 8 in die Atmosphäre entweicht. Der auf die Gummimembran 5 wirkende intrakranielle Druck Pw bewirkt eine leichte Auslenkung der Membran gegen den Öffnungsstutzen 3 und dadurch eine Änderung der Größe des Spaltes 7. Als Folge der Verengung des Spaltes 7 nimmt der Durchfluß der Luft durch den Öffnungsstutzen 3 und die flexible Leitung 8 in die Atmosphäre ab und der Druck Pm in der Kammer 6 ändert sich auf den Wert, bei welchem die auf die Gummimembran 5 durch den Druck Pw einwirkende Kraft mit der durch den Druck Pm erzeugten Kraft im Gleichgewicht ist. Der in der Überwachungseinrichtung abgelesene Wert des Druckes Pm ist ein Maß für den intrakraniellen Druck Pw.

## Patentansprüche

1. Pneumatischer Sensor für den intrakraniellen Druck zum Einsetzen zwischen den Schädelknochen und die Dura mater, mit einem Körper (1) aus Kunststoff in der Form einer flachen Dose und mit einer in diesem Körper angeordneten Meßeinrichtung, die mit einer Überwachungseinrichtung verbunden ist, dadurch gekennzeichnet, daß eine in dem Körper (1) ausgebildete kammer (6) über einen Spalt (7), einen Öffnungsstutzen (3) und eine mit ihrem einen Ende abgedichtet in den Körper (1) eingesetzte flexible Leitung (8) mit der Atmosphäre in Verbindung steht und über ein Loch (9) im Boden der Kammer (6) und eine mit einem Ende abgedichtet in dem Körper (1) eingesetzte flexible Leitung (10) mit einer Druckgasquelle in Verbindung steht und daß in dem oberen Teil (1b) des Körpers (1) eine ringförmige Rille (4) ausgebildet ist, in welche ein ringförmiger Rand (5a) einer Gummimembran (5) abdichtend eingesetzt ist.
